# EUROPÄISCHE PATENTSCHRIFT

(11) **EP 1 374 872 B1**
(45) Veröffentlichungstag und Bekanntmachung des Hinweises auf die Patenterteilung: **28.09.2005**
(21) Anmeldenummer: 03011966.3
(22) Anmeldetag: 28.05.2003
(51) Int. Cl.: A61K 31/573, A61K 31/405, A61K 31/192, A61K 31/655, A61K 31/65, A61K 31/7036, A61K 9/70, A61K 9/22, A61P 19/08, A61P 19/02, A61P 29/00, A61P 31/00, A61P 31/04

(54) **Pharmazeutische Zubereitungen mit einer Kombination aus jeweils mindestens einem Vertreter der Antibiotika und der Antiphlogistika, Verfahren zu deren Herstellung sowie deren Verwendung**
Pharmaceutical preparations comprising a combination of at least one representative of antibiotics and one of antiphlogistics, process for manufacturing, and use thereof
Les préparations pharmaceutiques comprenant une combination d' au moins un agent représentatif d'un antibiotique et un agent d'un antiphlogistique, le procès de leur préparation et leur utilisation

(30) Priorität: 21.06.2002 DE 10227938
(43) Veröffentlichungstag der Anmeldung: 02.01.2004
(73) Patentinhaber: Heraeus Kulzer GmbH, 63450 Hanau (DE)
(72) Erfinder: Vogt, Sebastian, Dr., 99084 Erfurt (DE); Schnabelrauch, Matthias, Dr., 07749 Jena (DE); Kühn, Klaus-Dieter, Dr., 35041 Marburg (DE)
(74) Vertreter: Kühn, Hans-Christian

(56) Entgegenhaltungen:
- EP-A- 0 420 600
- GB-A- 2 209 938
- US-A- 5 459 135
- BAEYENS V ET AL: "Optimized release of dexamethasone and gentamicin from a soluble ocular insert for the treatment of external ophthalmic infections" JOURNAL OF CONTROLLED RELEASE, ELSEVIER SCIENCE PUBLISHERS B.V. AMSTERDAM, NL, Bd. 52, Nr. 1-2, 2. März 1998 (1998-03-02), Seiten 215-220, XP004113669 ISSN: 0168-3659
- RENARD G ET AL: "Comparative study of a collagen corneal shield and a subconjunctival injection at the end of cataract surgery" JOURNAL OF CATARACT AND REFRACTIVE SURGERY 1993 UNITED STATES, Bd. 19, Nr. 1, 1993, Seiten 48-51, XP008020049 ISSN: 0886-3350
- RENARD G ET AL: "[Efficacy and tolerability of a combination of indomethacin and gentamicin for preventing inflammation after cataract surgery]" JOURNAL FRANCAIS D'OPHTALMOLOGIE. FRANCE 1996, Bd. 19, Nr. 11, 1996, Seiten 689-695, XP008020050 ISSN: 0181-5512
- VAN ENDT J J ET AL: "A comparison of two ophthalmic steroid-antibiotic combinations after cataract surgery." EUROPEAN JOURNAL OF OPHTHALMOLOGY. ITALY 1997 APR-JUN, Bd. 7, Nr. 2, April 1997 (1997-04), Seiten 144-148, XP008020051 ISSN: 1120-6721

## Beschreibung

Die vorliegende Erfindung betrifft pharmazeutische Zubereitungen, ihre Herstellung sowie deren Verwendung für die Human- und Veterinärmedizin zur Behandlung und Prävention lokaler bakterieller Infektionen.

Die Behandlung lokaler mikrobieller Infektionen des Weich- und Hartgewebes in der Human- und Veterinärmedizin erfordert hohe lokale Antibiotika-Konzentrationen im infizierten Gewebebereich. Es ist seit langem bekannt, dass eine systemische Anwendung von Antibiotika mit einer Reihe von Problemen behaftet ist. Bei der systemischen Anwendung ist es oft erforderlich, sehr hohe Antibiotika-Dosen einzusetzen, damit im infizierten Gewebe antimikrobiell wirksame Antibiotika-Konzentrationen erzielt werden. Dadurch kann es insbesondere bei den Aminoglucosid-Antibiotika auf Grund ihrer Nephro- und Ototoxizität zu schwerwiegenden Schädigungen des Organismus kommen. Daher lag der Gedanke nahe, Antibiotika in lokal applizierbaren Freisetzungssystemen einzusetzen, beziehungsweise sie in geeignete Depotformen zu überführen. Weiterhin ist allgemein bekannt, dass insbesondere lokale mikrobielle Infektionsprozesse mit ausgeprägten Entzündungsprozessen des infizierten Gewebes verbunden sind, die zu einer zusätzlichen Schädigung des infizierten Organismus führen können. Es ist daher von Vorteil, bei lokalen mikrobiellen Infektionen sowohl gegen die mikrobiellen Erreger antibiotisch vorzugehen und gleichzeitig die inflammatorischen Prozesse zu behandeln.

Depotsysteme zur verzögerten Freisetzung von antibiotischen Wirkstoffen für die Behandlung von lokalen Infektionen sind Gegenstand einer Vielzahl von Veröffentlichungen und Patenten. Neben physikalischen Retardsystemen, bei denen die Retardwirkung im wesentlichen auf Adsorptionseffekten und Diffusionsprozessen beruht, sind auch einige Retardsysteme auf Basis von geringlöslichen Antibiotika-Salzen und Antibiotika-Komplexen bekannt. Bisher fanden schwerlösliche Salze der Aminoglykosid-Antibiotika, der Tetracyclin-Antibiotika und der Lincosamid-Antibiotika relativ geringe Beachtung für die Herstellung von Depotpräparaten. Die Bildung von schwerlöslichen Salzen beziehungsweise Komplexen der Antibiotika des Tetracyclintyps sind seit Jahrzehnten allgemeiner Kenntnisstand. Es wurde zum Beispiel die Verwendung von Tetracylin-Sulfamaten zur antibiotischen Therapie vorgeschlagen (A. Jurando Soler, J. M. Puigmarti Codina: Antibiotic tetracycline sulfamate and ist derivatives. 27.10.1970, US 3,536,759; Anonym: Antibiotic tetracycline alkylsulfamates. 16.10.1969, ES 354 173; C. Ciuro, A. Jurado: Stability of a tetracycline derivative. Afinidad 28 (292) 1971, 1333-5.). Bei den Aminoglykosid-Antibiotika sind ebenfalls eine Reihe von schwerlöslichen Salzen prinzipiell bekannt. So wurde beim Gentamicin die Darstellung schwerlöslicher Salze basierend auf höheren Fettsäuren, Arylalkylcarbonsäuren, Alkylsulfaten und Alkylsulfonaten beschrieben (G. M. Luedemann, M. J. Weinstein: Gentamycin and method of production. 16.07.1962, US 3,091,572). Exemplarisch sind dafür Gentamicin-Salze der Laurinsäure, der Stearinsäure, der Palmitinsäure, der Ölsäure, der Phenylbuttersäure, der Naphthalen-1-carbonsäure, der Laurylschwefelsäure und der Dodecylbenzensulfonsäure. Diese Salze erwiesen sich vielfach als unvorteilhaft, weil sie wachsartige, hydrophobe Substanzen darstellen, die eine galenische Verwendung behindern. Trotzdem wurden Festtsäuresalze von Gentamicin und von Etamycin aus der freien Base beziehungsweise aus ihren Salzen in Wasser bei 50-80°C synthetisert (H. Voege, P. Stadler, H. J. Zeiler, S. Samaan, K. G. Metzger: Sapringly-soluble salts of aminoglycosides and formations containing them with inhibited substance-release. 28.12.1982, DE 32 48 328). Diese Antibiotika-Fettsäuresalze sollen als Injektionspräparate geeignet sein. Die Herstellung von Gentamicindodecylsulfat und dessen Verwendung in Salben und Cremes wurde ebenfalls beschrieben (A. Jurado Soler, J. Puigmarti Codina, J. A. Ortiz Hernandez: Neue Gentamicinderivate, Verfahren zur Herstellung derselben und diese enthaltende pharmazeutische Mittel. 21.04.1975, DE 25 17 600). Auch bei den Lincosamid-Antibiotika sind schwerlösliche Salze, wie zum Beispiel das Glindamycin-Palmitat bekannt (M. Cimbollek, B.Nies, R. Wenz, J. Kreuter: Antibiotic-impregnated heart valve sewing rings for treatment and prophylaxis of bacterial endocarditis. Antimicrob. Agents Chemother. 40(6) (1996)1432-1437). Eine neuere Entwicklung stellen schwerlösliche Aminoglykosid-Flavonoid-Phosphate dar (H. Wahlig, E. Dingeldein, R. Kirchlechner, D. Orth, W. Rogalski: Flavonoid phosphate salts of aminoglycoside antibiotics. 13.10.1986, US 4,617,293). Es werden die Salze der Phosphorsäurehalbester von Derivaten der Hydroxyflavane, Hydroxyflavene, Hydroxyflavanone, Hydroxyflavone und Hydroxyflavylium beschrieben. Besonders bevorzugt sind die Derivate der Flavanone und der Flavone. Diese schwerlöslichen Salze sollen als Depotpräparate Verwendung finden. So werden zum Beispiel diese Salze in Kollagenvliese eingebracht (H. Wahlig, E. Dingeldein, D. Braun:
Medicinally useful, shaped mass of collagen resorbable in the body. 22.09.1981, US 4,291,013).
Von V. Baevens et al. (Journal of Controlled Release 52 (1998) 215-220.) wurde ein Okularinsert beschrieben, das einen Formkörper darstellt, der durch Extrusion eines Gemisches aus einem hydrophoben und einem wasserlöslichen bioadhäsiven Polymer sowie einem Lösungsvermittler, Gentamicinsulfat und Dexamethasonphosphat gebildet wird.
Von G. Renard et al. (Cataract Refract Surg. 19 (1993) 48-51.) wurde eine Kollagen-Okulareinlage publiziert, die aus Kollagen besteht, die vor ihrer Verwendung in eine Lösung getaucht wurde, die Gentamicin und Dexamethason enthält.
Von G. Renard et. Al (J. Fr. Opthalmol. *19*, 11 (1996) 689-695.) wurden Augentropfen beschrieben, die folgende Kombinationen enthalten:
   a) 0,1 % Indomethacin/300 00 IE/10 ml Gentamicinsulfat
   b) 0,1 % Dexamethason/350 000 IE/100 ml Neomycinsulfat
Indomethacin kann als schwache Carbonsäure nicht die wesentlich stärker acide Schwefelsäure aus ihrem Gentamicinsalz verdrängen. Deshalb ist es nicht möglich, daß ein Gentamicin-Indomethacin-Salz aus Indomethacin und Gentamicinsulfat unter den offen gelegten Bedingungen entstanden ist. Die beschriebene Kombination aus Dexamethason und Neomycinsulfat ist auf Grund der nichtionischen Natur von Dexamethason nicht in der Lage gering lösliche antibiotische Salze zu bilden.

Bisher wurden keine Publikationen über antibiotisch-antiphlogistische Zubereitungen bekannt, die auf in Wasser gering löslichen Salzen beruhen, die aus einem Antiphlogistikum aus der Gruppe der Steroid-Antiphlogistika und der nichtsteroidalen Arylalkylcarbonsäuren und aus mindestens einem der Antibiotika Gentamicin, Clindamycin, Neomycin, Streptomycin, Tetracyclin, Doxicyclin, Oxytetracyclin und Rolitetracyclin aufgebaut sind.

Der vorliegenden Erfindung liegt die Aufgabe zugrunde, eine pharmazeutische Zubereitung als Antiphlogistika-Antibiotika-Kombination mit retardierender Wirkstofffreisetzung als Antibiotika-Depot für die Human- und Veterinärmedizin, zur Behandlung lokaler mikrobieller Infektionen im Knochen- und im Weichgewebe zu entwickeln.

Die Aufgabe wird erfindungsgemäß durch die Merkmale des Anspruchs 1 gelöst. Vorteilhafte Ausgestaltungen sind in den Unteransprüchen angegeben.

Gentamicin, Clindamycin, Neomycin, Streoptomycin, Tetracyclin, Doxicyclin, Oxytetracyclin und Rolitetracyclin sind häufig verwendete kationische Antibiotika, deren Aminogruppen in der Salzform protoniert sind und die mit üblichen Anionen, wie zum Beispiel Sulfat-Anionen und Chlorid-Anionen, in Wasser leicht lösliche Salze bilden. Die Alkalisalzformen der Antiphlogistika Ibuprofen, Naproxen, Indomethacin, Dexamethason-21-phosphat, Dexamethason-21-sulfat, Triamcinolon-21-phosphat und Triamcinolo-21-sulfat sind ebenfalls in Wasser leicht löslich.

Diese Kombination zeigt eine kontrollierte Antbiotika- und auch eine Antiphlogistika-Freisetzung im wäßrigen Milieu über einen Zeitraum von mehreren Tagen. Der Mechanismus der verzögerten Wirkstofffreisetzung ist im wesentlichen unabhängig von Trägermaterialien und beruht nicht auf Adsorptionseffekten an den Oberflächen von Trägermaterialien. Die Antiphlogistika-Antibiotika-Kombination kann unter Beibehaltung der Wirkstoffretardierung sowohl mit resorbierbaren als auch nichtresorbierbaren Hilfsstoffen unterschiedlichster Struktur zu Implantaten verarbeitet werden. Weiterhin ist die Art und Weise der Wirkstoffkombination nicht nur für ein spezielles Antibiotikum anwendbar, sondern sie ist vielmehr für eine Reihe Antibiotika ähnlicher Struktur geeignet.

Die Erfindung basiert auf dem überraschenden Befund, dass Salze, deren kationische Komponente ein Vertreter der Antibiotika Gentamicin, Clindamycin, Neomycin, Streoptomycin, TetraCyclin, Doxicyclin, Oxytetracyclin und Rolitetracyclin ist und deren anionische Komponente ein Vertreter der Antiphlogistika Ibuprofen, Naproxen, Indomethacin, Dexamethason-21-phosphat, Dexamethason-21-sulfat, Triamcinolon-21-phosphat und Triamcinolo-21-sulfat ist, in Wasser gering löslich sind.

Diese Salze können aus den wasserlöslichen Salzen der Antibiotika Gentamicin, Clindamycin, Neomycin, Streoptomycin, Tetracyclin, Doxicyclin, Oxytetracyclin und Rolitetracyclin und den wasserlöslichen Alkali-Salzen der Antiphlogistika lbuprofen, Naproxen, Indomethacin, Dexamethason-21-phosphat, Dexamethason-21-sulfat, Triamcinolon-21-phosphat und Triamcinolo-21-sulfat durch reziproken Salzaustausch gebildet werden. Die Herstellung kann durch Vermischung der in Wasser gelösten Antibiotika-Salze mit den in Wasser gelösten Antiphloistika-Salzen erfolgen, wobei die in Wasser gering löslichen antiphlogistischen Antibiotika-Salze als Feststoffe oder Öle ausfallen.

Es ist vorteilhaft, dass die Salze Gentamicin-Ibuprofen, Gentamicin-Naproxen, Gentamicin-Indomethacin, Gentamicin-Dexamethason-21-phosphat, Gentamicin-Triamcinolon-21-phosphat, Tetracyclin-Indomethacin, Tetracyclin-Indomethacin, Neomycin-Indomethacin, Clindamycin-Indomethacin, Streptomycin-Naproxen, Teracyclin-Naproxen, Clindamycin-Naproxen, Streoptomycin-Ibuprofen bevorzugt verwendet werden.

Weiterhin ist es zweckmäßig, dass die antiphlogistischen Antibiotika-Salze in Formkörper, Tabletten, Pulver, Granulate, Fäden, Gewirke, Gestricke und Vliese eingebracht werden, die erfindungsgemäß als permanente oder temporäre Implantate verwendet werden. Das bedeutet, dass die antiphloistischen Antibiotika-Salze in Tabletten, Pulvern, Granulaten, Fäden, Gewirken, Gestricken und Vliesen als pharmazeutisch aktive Wirkstoffkombinationen integriert werden.

Es ist zweckmäßig, dass die Salze Bestandteil von Beschichtungen sind, die auf Formkörper, Pulvern, Granulate, Fäden, Gewirke, Gestricke und Vliese aufgebracht werden, die erfindungsgemäß als permanente oder temporäre Implantate verwendet werden. Diese Beschichtungen können aus den antiphlogistischen Antibiotika-Salzen selbst bestehen oder aus einer Kombination mit einem polymeren Schichtbildner.

Der Erfindung liegt eine weiterer überraschender Befund zu Grunde, dass im festen Aggregatzustand befindliche Gemische, die aus mindestens einem in Wasser leicht löslichen Salz des Gentamicins, des Clindamycins, des Neomycins, des Streoptomycins, des Tetracyclins, des Doxicyclins, des Oxytetracyclins und des Rolitetracyclins und mindestens einem in Wasser leicht löslichen Salz des Ibuprofens, des Naproxens, des Indomethacins, des Dexamethason-21-phosphats, des Dexamethason-21-sulfates, des Triamcinolon-21-phosphates und des Triamcinolo-21-sulfates und mindestens einem anorganischen und/oder organischen pharmazeutischem Hilfsstoff aufgebaut sind, Antibiotika-Depots bilden. Diese Kombinationen können zum Beispiel zu Tabletten oder Formkörper gepresst werden. Es wurde überraschend gefunden, dass diese Tabletten und Formkörper eine verzögerte Wirkstofffreisetzung im wäßrigen Milieu zeigen. Dieser Befund kann darauf zurückgeführt werden, dass sich nach Einbringung der Tabletten und Formkörper in wäßriges Milieu durch Einwirkung von Wasser gering lösliche antiphlogistische Salze bilden. Das bedeutet, die kostenintensive Synthese der antiphlogistischen Antibiotika-Salze kann durch Verwendung von erfindungsgemäßen Gemischen aus mindestens einem in Wasser leicht löslichem Salz des Gentamicins, des Clindamycins, des Neomycins, des Streoptomycins, des Tetracyclins, des Doxicyclins, des Oxytetracyclins und des Rolitetracyclins und mindestens einem in Wasser leicht löslichen Salz des Ibuprofens, des Naproxens, des Indomethacins, des Dexamethason-21-phosphats, des Dexamethason-21-sulfates, des Triamcinolon-21-phosphates und des Triamcinolo-21-sulfates und mindestens einem anorganischen und/oder organischen pharmazeutischen Hilfsstoffes für die Tabletten- und Formkörperherstellung gespart werden. Dieser überraschende Befund ist wesentlich für eine extrem kostengünstige Tabletten- und Formkörperherstellung.

Der Gegenstand der vorliegenden Erfindung soll anhand der nachfolgenden Beispiele 1 bis 5 näher erläutert werden, ohne jedoch die Erfindung einzuschränken.

### Beispiel 1

### Herstellung von Gentamicin-Dexamethasonphosphat

Es werden 150 mg Gentamicinsulfat (AK 628) in 1 ml dest. Wasser gelöst und separat 120 mg Dexamethason-21-phosphat-Natriumsalz (Fluka) ebenfalls in 2 ml destilliertem Wasser gelöst. Anschließend wird unter Rühren die Dexamethason-21-phosphat-Natriumsalz-Lösung zur Gentamicinsulfat-Lösung getropft. Es fällt ein flockiger, schleimiger Niederschlag aus. Es wird Dexamethason-21-phosphat-Natriumsalz-Lösung weiterhin zugetropft bis kein weiter Niederschlag mehr ausfällt. Der Niederschlag wird mehrfach mit destilliertem Wasser gewaschen und anschließend bis zur Massekonstanz getrocknet.

**Ausbeute: 163 mg; Fp - 235 (Zersetzung); IR □ (cm**^{**-1**}**): 3600-3000 (□ OH); 2943 (□CH); 2871 (□ CH); 1716 (□ CO); 1664 (Aromat); 1620; 1466; 1394; 1302; 1245; 1100; 983; 890; 851; 529.**

### Beispiel 2

Es wird ein Gemisch aus 1000,0 mg Calciumsulfat-Dihydrat (Fluka), 250,0 mg Poly-L-lactid (M ∼ 10000 g/mol), 49,7 mg Gentamicinsulfat (AK 628) und 24,9 mg Dexamethason-21-phosphat-Natriumsalz (Fluka) vermahlen. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepresst.

### Beispiel 3

Es wird ein Gemisch aus 1000,0 mg Calciumsulfat-Dihydrat (Fluka), 250,0 mg Poly-L-lactid (M ∼ 10000 g/mol), 49,7 mg Gentamicinsulfat (AK 628) und 86,8 mg Naproxen-Natriumsalz (hergestellt durch Neutralisation von Naproxen (Fluka)) vermahlen. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepresst.

### Beispiel 4

Es wird ein Gemisch aus 1000,0 mg Calciumsulfat-Dihydrat (Fluka), 250,0 mg Poly-L-lactid (M ∼ 10000 g/mol), 49,7 mg Gentamicinsulfat (AK 628) und 78,5 mg Ibuprofen-Natriumsalz (hergestellt durch Neutralisation von Ibuprofen (Fluka)) vermahlen. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepresst.

### Beispiel 5

Es wird ein Gemisch aus 1000,0 mg Calciumsulfat-Dihydrat (Fluka), 250,0 mg Poly-L-lactid (M ∼ 10000 g/mol), 49,7 mg Gentamicinsulfat (AK 628) und 130,7 mg Indomethazin-Natriumsalz (hergestellt durch Neutralisation (Fluka)) vermahlen. Jeweils 200 mg dieses Gemischs werden mit einer Presse bei einem Druck von 5 Tonnen innerhalb von zwei Minuten zu scheibenförmigen Formkörpern, mit einem Durchmesser von 13 mm, gepresst.

### Antibiotika-Freisetzungsversuche

Die in den Beispielen 2-5 hergestellten Formkörper wurden in Sörensen-Puffer pH 7,4 eingebracht und in diesem bei 37°C über einen Zeitraum von zwei Wochen gelagert. Die Probennahme erfolgte täglich, wobei das Freisetzungsmedium ausgetauscht wurde. Die Antibiotika-Wertbestimmung wurde mit einem Agardiffusionstest unter Verwendung von Bacillus subtilis ATCC 6633 als Testkeim durchgeführt. Die Ergebnisse sind in der Tabelle dargestellt.

**Tab.:**

| Kumulierte Gentamicin-Freisetzung (als Gentamicin-Base) aus Probekörpern der Beispiele 2-5 in Abhängigkeit von der Lagerungszeit im Sörensen-Puffer bei 37°C. | | | | | | | | |
|---|---|---|---|---|---|---|---|---|
| Beispiele | kumulierte Gentamicin-Freisetzung (als Gentamicin-Base) [mg] | | | | | | | |
| | Lagerungszeit [d] | | | | | | | |
| | 1 | 2 | 3 | 4 | 5 | 6 | 7 | 8 |
| 2 | 2,80 | 3,22 | 3,46 | 3,60 | 3,74 | 3,83 | 3,90 | 3,95 |
| 3 | 2,08 | 3,05 | 3,61 | 3,86 | 3,90 | 3,92 | 3,93 | 3,93 |
| 4 | 1,64 | 2,03 | 2,36 | 2,71 | 3,20 | 3,40 | 3,59 | 3,82 |
| 5 | 2,83 | 3,22 | 3,31 | 3,34 | 3,34 | 3,45 | 3,45 | 3,45 |

## Patentansprüche

1. Pharmazeutische Zubereitung, **dadurch gekennzeichnet, dass** sie Salze, deren kationische Komponente mindestens ein Vertreter der Antibiotika Gentamicin, Clindamycin, Neomycin, Streptomycin, Tetracyclin, Doxicyclin, Oxytetracyclin und Rolitetracyclin ist und deren anionische Komponente mindestens ein Vertreter der Antiphlogistika Ibuprofen, Naproxen, Indomethacin, Dexamethason-21-phosphat, Dexamethason-21-sulfat, Triamcinolon-21-phosphat und Triamcinolon-21-sulfat ist, enthält.

2. Pharmazeutische Zubereitung nach Anspruch 1, **dadurch gekennzeichnet, dass** sie die Salze Gentamicin-Ibuprofen, Gentamicin-Naproxen, Gentamicin-Indomethacin, Gentamicin-Dexamethason-21-phosphat, Gentamicin-Triamcinolon-21-phosphat, Tetracyclin-Indomethacin, Tetracyclin-Indomethacin, Neomycin-Indomethacin, Clindamycin-Indomethacin, Streptomycin-Naproxen, Tetracyclin-Naproxen, Clindamycin-Naproxen, Streptomycin-Ibuprofen enthält.

3. Pharmazeutische Zubereitung nach Anspruch 1 oder 2, **dadurch gekennzeichnet, dass** die Salze in Formkörper, Tabletten, Pulver, Granulate, Fäden, Gewirke, Gestricke und Vliese eingebracht sind.

4. Pharmazeutische Zubereitung nach Anspruch 1 oder 2, **dadurch gekennzeichnet, dass** die Salze Bestandteil von Beschichtungen sind, die auf Formkörper, Pulvern, Granulate, Fäden, Gewirke, Gestricke und Vliese aufgebracht sind.

5. Verwendung der pharmazeutischen Zubereitung nach Anspruch 1 zur Herstellung von Antibiotika-Depots.

6. Verwendung der pharmazeutischen Zubereitung nach einem der Ansprüche 3 und 4 zur Herstellung von permanenten oder temporären Implantaten.

## Claims

1. Pharmaceutical preparation, **characterized in that** it contains salts, the cationic component of which is at least one member of the antibiotics gentamicin, clindamycin, neomycin, streptomycin, tetracycline, doxicycline, oxytetracycline, rolitetracycline and the anionic component of which is at least one member of the antiphlogistic agents ibuprofen, naproxen, indomethacin, dexamethasone 21-phosphate, dexamethasone 21-sulphate, triamcinolone 21-phosphate and triamcinolone 21-sulphate.

2. Pharmaceutical preparation according to Claim 1, **characterized in that** it contains the salts gentamicin ibuprofen, gentamicin naproxen, gentamicin indomethacin, gentamicin dexamethasone 21-phosphate, gentamicin triamcinolone 21-phosphate, tetracycline indomethacin, tetracycline indomethacin, neomycin indomethacin, clindamycin indomethacin, streptomycin naproxen, tetracycline naproxen, clindamycin naproxen or streptomycin ibuprofen.

3. Pharmaceutical preparation according to Claim 1 or 2, **characterized in that** the salts are introduced into mouldings, tablets, powders, granules, filaments, machine knitted fabrics, knitted fabrics and nonwovens.

4. Pharmaceutical preparation according to Claim 1 or 2, **characterized in that** the salts are a component of coatings which are applied to mouldings, powders, granules, filaments, machine knitted fabrics, knitted fabrics and nonwovens.

5. Use of the pharmaceutical preparation according to Claim 1 for the production of antibiotic depots.

6. Use of the pharmaceutical preparation according to either of Claims 3 and 4 for the production of permanent or temporary implants.

## Revendications

1. Composition pharmaceutique, **caractérisée en ce qu'**elle contient des sels dont le constituant cationique est au moins un membre des antibiotiques gentamycine, clindamycine, néomycine, streptomycine, tétracycline, doxycycline, oxytétracycline et rolitétracycline et dont le constituant anionique est au moins un membre des antiphlogistiques ibuprofène, naproxène, indométhacine, dexaméthasone-21-phosphate, dexaméthasone-21-sulfate, triamcinolone-21-phosphate et triamcinolone-21-sulfate.

2. Composition pharmaceutique selon la revendication 1, **caractérisée en ce qu'**elle contient les sels gentamycine-ibuprofène, gentamycine-naproxène, gentamycine-indométhacine, gentamycine-dexaméthasone-21-phosphate, gentamycine-triamcinolone-21-phosphate, tétracycline-indométhacine, tétracycline-indométhacine, néomycine-indométhacine, clindamycine-indométhacine, streptomycine-naproxène, tétracycline-naproxène, clindamycine-naproxène, streptomycine-ibuprofène.

3. Composition pharmaceutique selon la revendication 1 ou 2, **caractérisée en ce que** les sels sont introduits dans des corps moulés, des comprimés, des poudres, des granulés, des fils, des tissus à mailles et des non-tissés.

4. Composition pharmaceutique selon la revendication 1 ou 2, **caractérisée en ce que** les sels sont des constituants de revêtements qui sont appliqués sur des corps moulés, des poudres, des granulés, des fils, des tissus à mailles et des non-tissés.

5. Utilisation de la composition pharmaceutique selon la revendication 1 pour la préparation de compositions d'antibiotiques à effet retard.

6. Utilisation de la composition pharmaceutique selon l'une des revendications 3 et 4 pour la préparation d'implants permanents ou temporaires.
